# EUROPÄISCHE PATENTSCHRIFT

(11) **EP 2 436 959 B1**
(45) Veröffentlichungstag und Bekanntmachung des Hinweises auf die Patenterteilung: **14.05.2014**
(21) Anmeldenummer: 10075607.1
(22) Anmeldetag: 01.10.2010
(51) Int. Cl.: F16K 11/085, A61M 1/10

(54) **Ventil, Pumpensystem und Verfahren zum Betrieb eines Pumpensystems**
Valve, pump system and method for operating a pump system
Soupape, système de pompe et procédé de fonctionnement d'un système de pompe

(43) Veröffentlichungstag der Anmeldung: 04.04.2012
(73) Patentinhaber: Berlin Heart GmbH, 12247 Berlin (DE)
(72) Erfinder: Hering, Jörg, 15370 Fredersdorf (DE); Schwiertz, Norbert, 15344 Strausberg (DE)
(74) Vertreter: Pfenning, Meinig & Partner GbR

(56) Entgegenhaltungen:
- WO-A2-89/01600
- US-A- 5 232 434

## Beschreibung

Die Erfindung liegt auf dem Gebiet des Maschinenbaus und befasst sich speziell mit Fluidventilen und hydraulischen bzw. pneumatischen Pumpensystemen, die mittels derartiger Ventile gesteuert werden.

Ventile sind zur Öffnung und zum Schließen von Fluidleitungen bzw. zur Steuerung von hydraulischen und/oder pneumatischen Kreisen in vielfältigen Ausführungsformen bekannt. Dabei unterscheiden sich verschiedene Ventile durch ihre Belastbarkeit, durch die Geschwindigkeit des Ansprechens bzw. der Betätigungsmöglichkeit, die Dichtigkeit, Druckbelastbarkeit und die Zahl der Schaltspiele, die ohne nennenswerten Verschleiß durchlaufen werden können. Die Ausgestaltung derartiger Ventile in Bezug auf die Materialwahl, die Konstruktion sowie die zulässigen Fertigungstoleranzen hängt von den individuellen Anforderungen ab.

Bei Fluidkreisen im Druckbereich von wenigen Atmosphärenüberdruck werden beispielsweise Schiebeventile betätigt, bei denen ein Schieber in einer Bohrung eines Anschlussblocks translatorisch bewegbar ist und mittels Ausnehmungen, die im Schieber vorgesehen sind, verschiedene Öffnungen bzw. Kanäle des Anschlussblocks in Abhängigkeit von der Schiebestellung miteinander verbindet. Dadurch können durch ein derartiges Ventil wahlweise verschiedene Fluidleitungen druckbeaufschlagt werden.

Die Spieltoleranz bei den Maßen eines derartigen Schiebers im Verhältnis zu der Bohrung, in der er sich bewegt, kann beispielsweise in der Größenordnung von hundertstel Millimetern liegen, um unzulässige Leckagen zu vermeiden. Es kann sich bei der Bewegung eines derartigen Schiebers ein Luftkisseneffekt bei richtiger Bemessung der Toleranzen ausbilden, der direkte Berührung und Abrieb vermeidet.

Aus der deutschen Patentschrift DE 10 2006 011 580 B3 der Firma Numatics GmbH ist zudem ein Drehschiebeorgan zur pneumatischen Steuerung bekannt geworden, das zur Druckluftsteuerung dient und einen in einer Zylinderbohrung drehbaren Steuerschieber vorsieht, der drehbar und antreibbar ist, wobei der Steuerschieber Oberflächennuten oder Innenkanäle aufweist, die drehstellungsabhängig Querbohrungen in dem Anschlussblock miteinander verbinden oder voneinander trennen. Dabei ist zwischen der Zylinderbohrung in dem Anschlussblock und dem Steuerschieber ein Zylinderspalt so bemessen, dass eine reibungsarme Lagerung nach dem Luftlagerprinzip gegeben ist.

Es wird aus der zitierten Patentschrift deutlich, dass mittels des Schiebers jeweils verschiedene Kanäle im Anschlussblock miteinander verbunden werden, die auf unterschiedlichen axialen Höhen in Bezug auf die Drehachse des Schiebers angeordnet sind.

Oft stellt sich die Aufgabe, pneumatische Kreise derart umzuschalten, dass einerseits in den verschiedenen Schaltstellungen der pneumatische Widerstand der Leitungen und Ventile minimiert wird und dass andererseits bei verschiedenen Schaltstellungen möglichst gleichbleibende pneumatische Strömungswiderstände eingehalten werden.

Der vorliegenden Erfindung liegt die Aufgabe zugrunde, ein Ventil zu schaffen, das schnelle und effiziente Umschaltmöglichkeiten mit einem möglichst minimierten Strömungswiderstand und einfachem konstruktivem Aufbau des Ventils verbindet.

Die Aufgabe wird mit den Merkmalen der Erfindung gemäß Patentanspruch 1 gelöst.

Dazu ist gemäß der Erfindung bei einem Ventil zur Steuerung eines Fluidflusses ein Anschlussblock vorgesehen, der eine zylindersymmetrische Ausnehmung aufweist, wobei in der Mantelfläche der Ausnehmung mindestens ein erster und ein zweiter Eingangskanal sowie ein erster und ein zweiter Ausgangskanal münden. Zudem ist ein in die Ausnehmung eingepasster, um eine Drehachse drehbarer Steuerzapfen vorgesehen, der wenigstens zwei Durchgangskanäle aufweist und in Abhängigkeit von seiner Winkelstellung verschiedene der Eingangs- und Ausgangskanäle des Anschlussblocks miteinander verbindet. Dazu ist in dem Zapfen ein erster Durchgangskanal vorgesehen, der bezüglich der Drehachse ausschließlich in einer ersten axialen Höhe mündende Kanäle des Anschlussblocks miteinander verbindet, sowie ein zweiter Durchgangskanal, der ausschließlich in einer zweiten axialen Höhe mündende Kanäle des Anschlussblocks miteinander verbindet.

Auf diese Weise ist sichergestellt, dass in den verschiedenen Schaltstellungen des Zapfens die Durchgangskanäle jeweils ausschließlich auf einer einzigen axialen Höhe in Bezug auf die Drehachse verlaufen und dass damit verschiedene durchzuschaltende Kanäle keine Kanalabschnitte aufweisen, die in Längsrichtung (Richtung der Drehachse) des Zapfens verlaufen, so dass die Richtungsänderungen der Durchgangskanäle minimiert sind. Hierdurch wird der Strömungswiderstand des Ventils in den verschiedenen Stellungen minimiert. Außerdem sind auch die in den Zapfen einzuarbeitenden Durchgangskanäle konstruktiv einfach zu realisieren, da sie keine in Längsrichtung des Zapfens verlaufende Abschnitte enthalten müssen. Sie können damit beispielsweise als einfache Querbohrungen in den Zapfen realisiert sein.

Da hierdurch auch die Länge der Durchgangskanäle kurz gehalten werden kann, sind auch Totvolumina beim Umschalten gering gehalten.

Ein weiterer Vorteil derartiger durch Drehung eines Zapfens betätigbarer Ventile besteht darin, dass die Anwendung des zu steuernden Drucks keine Krafteinwirkung auf die bewegbaren Elemente des Ventils entfaltet. Eine Tendenz des Ventils, sich durch den wirkenden Druck zu verstellen, ist somit nicht gegeben.

Eine vorteilhafte Ausgestaltung der Erfindung sieht vor, dass wenigstens ein Durchgangskanal, insbesondere der erste und der zweite Durchgangskanal, quer durch den Zapfen zwischen einer ersten und einer zweiten diametral einander gegenüberliegenden Zapfenöffnung verläuft und einen Abzweigkanal aufweist, der an einer dritten Zapfenöffnung endet.

Ein Teil des Durchgangskanals kann somit einfach durch Einbringen einer Querbohrung in den Zapfen eingebracht werden. Ein Abzweigkanal, der einen weiteren Teil des Durchgangskanals bildet, kann zudem einfach dadurch eingebracht werden, dass eine Radialbohrung in den Zapfen eingebracht wird, die von seiner Mantelfläche senkrecht zur Längsachse so tief eingebracht wird, dass sie auf den beispielsweise mittig quer durch den Zapfen verlaufenden Kanal stößt.

Damit ergibt sich eine T-förmige oder Y-förmige Kanalführung des Durchgangskanals, wobei alle Schenkel des Durchgangskanals in derselben axialen Höhe auf einer Querschnittsebene des Zapfens verlaufen und in drei Öffnungen an der Mantelfläche des Zapfens münden, von denen beispielsweise zwei einander diametral am Umfang des Zapfens gegenüberliegen und eine dritte seitlich symmetrisch oder asymmetrisch zwischen den erstgenannten beiden Öffnungen liegt.

Dabei richtet sich die Verteilung der Öffnungen des Durchgangskanals nach der Anordnung der entsprechenden Mündungen von Eingangs- und Ausgangskanälen bzw. weiteren Kanälen in der inneren Mantelfläche der Ausnehmung in dem Anschlussblock.

Eine weitere vorteilhafte Ausgestaltung der Erfindung sieht vor, dass in dem Anschlussblock wenigstens ein erster, in der ersten axialen Höhe in der Mantelfläche der Ausnehmung mündender Umschaltkanal vorgesehen ist, der diese Mündung mit einem in der zweiten axialen Höhe mündenden Kanal verbindet.

In dem Anschlussblock ist somit auf der ersten axialen Höhe die Mündung eines Eingangskanals, die Mündung eines Ausgangskanals und die Mündung eines Umschaltkanals vorgesehen, wobei die drei genannten Kanäle in verschiedenen Drehstellungen des Steuerzapfens miteinander verbunden werden können. In einer Schaltstellung kann dabei der Eingangskanal mit dem Ausgangskanal verbunden werden, wodurch das Ventil eine Fluidleitung zwischen dem Eingangskanal und dem Ausgangskanal gerade durchverbindet.

In einer anderen Schaltstellung des Zapfens kann der entsprechende Eingangskanal mit einem Umschaltkanal und über diesen mit einem zweiten Ausgangskanal verbunden werden, der in einer zweiten axialen Höhe innerhalb der Mantelfläche des Anschlussblocks mündet.

Damit kann der erste Eingangskanal auf einen zweiten Ausgangskanal durchgeschaltet werden. Insgesamt ermöglicht diese Konfiguration ein Durchschalten eines Eingangskanals auf zwei Ausgangskanäle.

Zudem kann vorteilhaft vorgesehen sein, dass in dem Anschlussblock wenigstens ein zweiter, in der zweiten axialen Höhe in der Mantelfläche der Ausnehmung mündender Umschaltkanal vorgesehen ist, der diese Mündung mit einem in der ersten axialen Höhe mündenden Kanal verbindet. Damit ist ermöglicht, dass mittels des Zapfens in der zweiten axialen Höhe der zweite Eingangskanal entweder auf den zweiten Ausgangskanal durchgeschaltet werden kann oder dass der zweite Eingangskanal mit einem in der zweiten axialen Höhe mündenden Umschaltkanal verbunden werden kann, der seinerseits mit einem in der ersten axialen Höhe mündenden Kanal verbunden ist. Dieser in der ersten axialen Höhe mündende Kanal kann beispielsweise der erste Ausgangskanal sein, so dass in einer geeigneten Schaltstellung der zweite Eingangskanal mit dem ersten Ausgangskanal verbunden werden kann.

In einer besonders vorteilhaften Ausgestaltung der Erfindung kann vorgesehen sein, dass der Zapfen einen dritten Durchgangskanal aufweist.

Dabei kann zudem vorgesehen sein, dass der dritte Durchgangskanal in Abhängigkeit von der Winkelstellung des Zapfens zwei Abschnitte eines Umschaltkanals miteinander verbindet.

Es ist damit ermöglicht, nicht nur durch geeignete Stellungen des Zapfens die Umschaltkanäle zu nutzen und damit eine Überkreuzzuordnung der Eingangskanäle zu den Ausgangskanälen herzustellen, sondern zusätzlich wenigstens einen der Umschaltkanäle gleichzeitig dadurch zu schalten, dass dieser abschnittsweise durch den Zapfen verläuft. Damit ist es möglich, in den Schaltstellungen, in denen die Eingangskanäle jeweils gerade durchgeschaltet werden, wenigstens einen der Umschaltkanäle zu schließen und damit ein Überströmen zwischen den zu schaltenden Kanälen zu verhindern.

Der dritte Umschaltkanal ist vorteilhaft auf einer axialen Höhe zwischen der ersten und der zweiten axialen Höhe innerhalb des Zapfens als Querbohrung vorgesehen.

Die Erfindung kann außerdem vorteilhaft dadurch ausgestaltet werden, dass der Anschlussblock einen Ventilhohlzylinder aufweist, der den Zapfen aufnimmt und Dichtungen zum Abdichten der Kanäle zwischen dem Zapfen und dem Anschlussblock aufweist.

Der Anschlussblock ist demgemäß mit einem Ventilhohlzylinder versehen, der einen Einsatz bildet und seinerseits an seiner inneren Mantelfläche die Ausnehmung für den Steuerzapfen bildet. Der Ventilhohlzylinder kann Dichtungselemente wie beispielsweise Elastomerdichtungen an seiner inneren Mantelfläche aufweisen, die in den verschiedenen Winkelstellungen des Zapfens die jeweils zu verbindenden Kanalmündungen abdichten.

Beispielsweise kann der Ventilhohlzylinder auch umlaufende Elastomerdichtungen in Umfangsrichtung des Zapfens aufweisen, die grundsätzlich die Gruppen von Kanalmündungen auf verschiedenen axialen Höhen bezüglich der Drehachse des Zapfens gegeneinander abschotten. Es können anstelle von Elastomerdichtungen auch andere Dichtungen vorgesehen sein, die bezüglich der Maßtoleranz derart ausgebildet sind, dass eine effiziente Abdichtung bei geringer Reibungsentwicklung gegeben ist. Das Ventil soll im Idealfall in der Lage sein, mit Reaktionszeiten im Millisekundenbereich durch Änderung der Winkelstellung des Zapfens verschiedene pneumatische Kanäle zu schalten.

Der separate Ventilhohlzylinder hat den Vorteil, dass er separat gut bearbeitbar ist und mit Einsätzen aus anderen Materialien versehen werden kann, bevor er in den Anschlussblock eingesetzt wird. Der Ventilhohlzylinder ist im Betrieb mit den übrigen Teilen des Anschlussblocks unbeweglich verbunden. Er weist lediglich radial durchgehende Öffnungen auf, die jeweils einen Teil der Eingangs-, Ausgangs- und Umschaltkanäle bilden.

Der übrige Teil des Anschlussblocks weist die Fortsetzungen der Eingangs- und Ausgangskanäle sowie Umschaltkanäle auf, die wenigstens teilweise auch in Längsrichtung des Steuerzapfens verlaufen, um die Verbindungen zwischen den in der ersten Höhe und in der zweiten Höhe mündenden Kanälen herzustellen.

Dieser Teil des Anschlussblocks kann als massiver, von gefrästen Kanälen durchsetzter Block ausgebildet sein und beispielsweise aus einem Metall bestehen. Auch die Herstellung aus einem stabilen Kunststoff oder einer Keramik ist möglich.

Um den Steuerzapfen rotatorisch antreibbar zu machen, ist gemäß der Erfindung vorteilhaft vorgesehen, dass der Steuerzapfen an einem seiner Enden mit einem Magneten verbunden ist, der einen Teil eines Drehantriebes bildet. Dabei kann der Steuerzapfen in einem Formkörper enden, auf den ein Dauermagnet aufgesetzt ist, der mit einem schaltbaren Elektromagneten in Abhängigkeit des Magnetfeldes des Elektromagneten seine verschiedenen Schaltstellungen einnimmt.

Zudem kann vorteilhaft vorgesehen sein, dass mechanische Anschläge zwischen dem Zapfen und dem Anschlussblock vorgesehen sind, die zwei Endwinkelstellungen des Zapfens definieren.

Durch mechanische Anschläge werden die Drehstellungen/Winkelstellungen des Steuerzapfens genügend genau definiert, um die Mündungen der Kanäle in den Mantelflächen des Zapfens und der Ausnehmung ausreichend genau in Deckung zu bringen und damit den pneumatischen Widerstand gering zu halten. Damit werden die Anforderungen an die Genauigkeit des Antriebs bei der Einstellung der verschiedenen Winkelstellungen reduziert.

Es können zwischen den durch Anschläge definierten Endstellungen des Zapfens auch bevorzugte Zwischenstellungen dadurch eingestellt werden, dass diese durch eine Kugelrast zwischen dem Zapfen und dem Anschlussblock gekennzeichnet und stabilisiert sind.

Die Erfindung bezieht sich außer auf ein Ventil der oben beschriebenen Art zudem auf ein Pumpensystem mit zwei Antriebspumpen, die mittels Fluidleitungen mit jeweils einer Arbeitspumpe zur Förderung einer Flüssigkeit, insbesondere Blut verbindbar sind, wobei beide Fluidleitungen ein gemeinsames Ventil der oben beschriebenen Art durchlaufen und das Ventil als 2x2-Wege-Kreuzventil ausgebildet ist.

Die Antriebspumpen können dabei beispielsweise als Membranpumpen ausgebildet sein, die durch einen wechselnden Fluiddruck einer Arbeitspumpe wechselnde Membranstellungen einnehmen und dadurch eine pulsatile Pumpwirkung entfalten. Auf diesem Prinzip beruhen beispielsweise Membranpumpen, die im menschlichen Körper als Blutpumpen einsetzbar sind und die Tätigkeit des Herzens unterstützen können. Die Einstellung der Membran erfolgt über die Steuerung eines Sekundärdrucks mittels einer Arbeitspumpe, die als pneumatische Pumpe ausgebildet und mittels einer Fluidleitung mit der jeweiligen Antriebspumpe verbunden ist. Im biventrikulären Betrieb derartiger Pumpen werden zwei Antriebspumpen benötigt, die an verschiedenen Herzkammern ansetzen und mit jeweils einer Arbeitspumpe verbunden sind.

Fällt eine der Arbeitspumpen aus, so kann mittels eines 2x2-Wege-Kreuzventils die verbleibende intakte Arbeitspumpe wechselweise auf die zwei Antriebspumpen durchgeschaltet werden, so dass beide Herzkammern noch unterstützt werden können.

Die Erfindung bezieht sich insofern auch auf ein Verfahren zum Betrieb eines Pumpensystems mit einem Ventil der beschriebenen Art, das mit wenigstens einer arbeitsfähigen Antriebspumpe und mit zwei Arbeitspumpen verbunden und als 2x2-Wege-Kreuzventil ausgebildet ist, dadurch gekennzeichnet, dass das Ventil eine Antriebspumpe abwechselnd mit je einer der Arbeitspumpen verbindet.

Im Folgenden wird die Erfindung anhand eines Ausführungsbeispiels in einer Zeichnung gezeigt und nachfolgend beschrieben.

Dabei zeigt
- Fig. 1: schematisch ein Pumpensystem mit zwei Antriebspumpen und zwei Arbeitspumpen, die als Blutpumpen bei einem menschlichen Patienten eingesetzt sind,
- Fig. 2: eine detailliertere Darstellung einer Membranarbeitspumpe,
- Fig. 3: eine schematische Darstellung der Kanäle in einem Ventil,
- Fig. 4: eine schematische Darstellung gekreuzter Kanäle in einem Ventil,
- Fig. 5: eine schematische Darstellung eines Ventils mit einem durchgehenden und einem gesperrten Kanal,
- Fig. 6: eine schematische Darstellung eines Ventils mit einem umgeschalteten Kanal,
- Fig. 7: eine schematische Darstellung eines Ventils mit einem zweiten durchgehenden Kanal, wobei der erste Kanal gesperrt ist,
- Fig. 8: eine schematische Darstellung eines Ventils, wobei der zweite Kanal umgeschaltet ist,
- Fig. 9: eine dreidimensionale Ansicht eines Anschlussblocks,
- Fig. 10: drei dreidimensionale Ansichten desselben Ventilzylinders aus verschiedenen Blickwinkeln,
- Fig. 11: drei dreidimensionale Ansichten eines Steuerzapfens aus verschiedenen Blickwinkeln,
- Fig. 12: schematisch untereinander drei Querschnitte eines Ventils mit einem Steuerzapfen auf drei verschiedenen axialen Höhen, in denen Durchgangskanäle im Steuerzapfen, Einlass- und Auslasskanäle angeordnet sind,
- Fig. 13: die Darstellung aus Fig. 12 mit gleichliegenden Querschnitten, wobei der Steuerzapfen um 90° gegenüber der Darstellung in Fig. 12 im Uhrzeigersinn gedreht ist,
- Fig. 14: eine teilweise durchbrochene Ansicht des Anschlussblocks mit dem darin sichtbaren Steuerzapfen,
- Fig. 15: eine schematische Ansicht des Steuerzapfens mit symbolisch dargestellten Umschaltkanälen sowie
- Fig. 16: Die Anordnung aus Fig. 15, aus der entgegengesetzten Blickrichtung dargestellt.

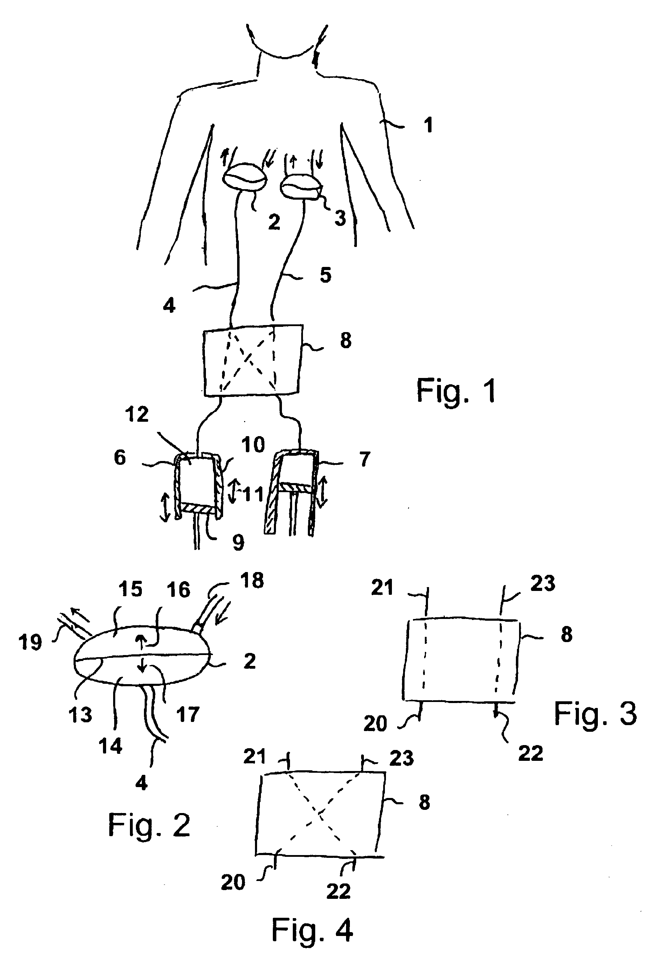

Fig. 1 zeigt symbolisch die Silhouette 1 eines Menschen mit zwei Membranpumpen 2, 3, von denen jeweils eine mit einem Blutgefäß verbunden ist, sowie zwei entsprechende pneumatische Zuleitungen 4, 5, über die die Membranpumpen 2, 3 mit je einer Antriebspumpe 6, 7 verbunden sind. Schematisch ist weiterhin ein Ventil 8 dargestellt, das als 2x2-Wege-Kreuzventil ausgebildet ist.

In einer ersten Schaltstellung verbindet das Ventil 8 die Antriebspumpe 6 mit der Arbeitspumpe 2 und die Antriebspumpe 7 mit der Arbeitspumpe 3.

In einem zweiten Schaltzustand verbindet das Ventil 8 die Antriebspumpe 6 mit der Arbeitspumpe 3 und die Antriebspumpe 7 mit der Arbeitspumpe 2.

Durch diese Schaltmöglichkeit kann im biventrikulären Betrieb, wenn durch je eine der Arbeitspumpen 2, 3 die Funktion einer Herzkammer unterstützt wird, das gesamte System mit nur einer Antriebspumpe 6, 7 betrieben werden, falls eine der Antriebspumpen 6, 7 ausfällt. In diesem Fall wird die verbleibende intakte Antriebspumpe 6, 7 wechselweise mittels des Ventils 8 auf die Arbeitspumpen 2, 3 umgeschaltet, so dass jede der Arbeitspumpen etwa zwei Herzschläge unterstützen kann bzw. zwei pulsatile Perioden durcharbeitet.

Die Antriebspumpen 6, 7 sind als pneumatische Kolbenpumpen mit je einem Kolben 9 und einem Zylinder 10 ausgebildet, wobei der Kolben 9 mittels eines nicht näher dargestellten Antriebs in Richtung des Pfeils 11 wechselweise antreibbar ist, um in dem Antriebsvolumen wechselnd Über- und Unterdruck zu erzeugen, der mittels der pneumatischen Leitung 4, 5 jeweils zu einer der Membranpumpen 2, 3 geleitet wird.

In der Fig. 2 ist detaillierter der Aufbau einer Membranpumpe 2 beschrieben. Diese weist eine flexible Membran 13 auf, die einen Antriebsraum 14 von einem Arbeitsraum 15 trennt. Die Membran 13 ist flexibel und mit einem Stoff beschichtet, der die Koagulation von Blut verhindert.

Der Antriebsraum 14 ist direkt mit einer Zuleitung 4 verbunden, die durch Zu- und Ableitung eines Gases, insbesondere Luft, dafür sorgt, dass der Antriebsraum 14 sich wechselweise vergrößert und verkleinert und damit die Membran sich in Richtung der Pfeile 16, 17 bewegt.

Der Arbeitsraum 15 verkleinert sich zwangsweise, wenn der Antriebsraum 14 sich vergrößert, und umgekehrt. Dadurch wird in den Arbeitsraum 15 mittels der Blutzuleitung 18 Blut eingesaugt, und bei Verkleinerung des Arbeitsvolumens 15 wird dies durch die Blutableitung 19 wieder ausgestoßen. Einwegrückschlagventile in der Zuleitung 18 und der Ableitung 19 sorgen dafür, dass der Blutstrom eindeutig gerichtet ist.

Die Fig. 3 zeigt eine schematische Darstellung des Ventils 8, in der ein erster Einlasskanal 20 mit einem ersten Auslasskanal 21 und ein zweiter Einlasskanal 22 mit einem zweiten Auslasskanal 23 verbunden ist. Der Einlasskanal 20 kann mit der Arbeitspumpe 6, der Auslasskanal 21 mit der Arbeitspumpe 2 verbunden sein, während der Einlasskanal 22 mit der Antriebspumpe 7 und der Auslasskanal 23 mit der Antriebspumpe 3 verbunden ist.

Fig. 4 zeigt einen anderen Schaltzustand des Ventils 8, in dem der erste Einlasskanal 20 mit dem zweiten Auslasskanal 23 und der zweite Einlasskanal 22 mit dem ersten Auslasskanal 21 verbunden ist, wie durch die gestrichelt dargestellten Umschaltkanäle symbolisiert ist.

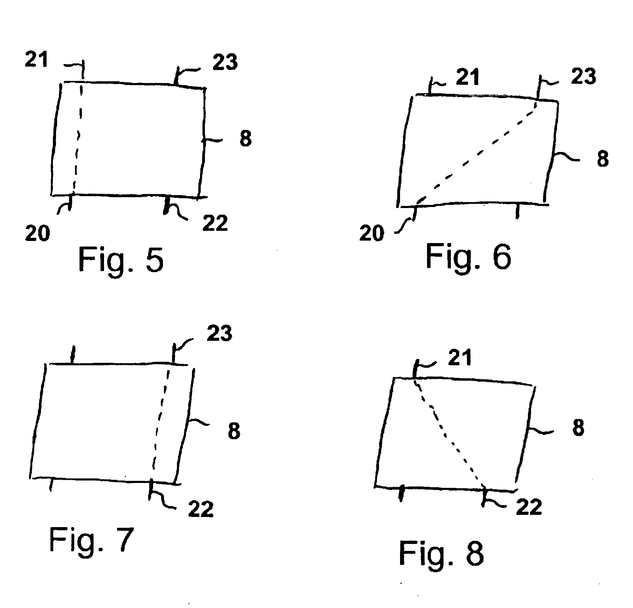

Fig. 5 zeigt eine Darstellung, in der der erste Einlasskanal 20 mit dem ersten Auslasskanal 21 verbunden ist, wobei die Fluidförderung vom zweiten Einlasskanal 22 zum zweiten Auslasskanal 23 nicht dargestellt ist, da die Antriebspumpe 7 als nicht funktionsfähig vorausgesetzt wird. Es strömt damit ausschließlich Gas vom ersten Einlasskanal 20 zum ersten Auslasskanal 21 und zurück, und es wird nur die Arbeitspumpe 2 betrieben.

Fig. 6 zeigt den umgeschalteten Zustand des Ventils 8, in dem der erste Einlasskanal 20 mit dem zweiten Auslasskanal 23 und damit über die Zuleitung 5 mit der zweiten Arbeitspumpe 3 verbunden ist, so dass mittels der Antriebspumpe 6 die zweite Arbeitspumpe 3 versorgt werden kann.

Fig. 7 zeigt einen alternativen Fall, in dem die Antriebspumpe 7 intakt ist und über das Ventil 8 mittels des Einlasskanals 22 über den Auslasskanal 23 mit der Arbeitspumpe 3 verbunden werden kann.

Dieser Zustand führt nach Umschalten des Ventils 8, wie in der Fig. 8 dargestellt, dazu, dass der zweite Einlasskanal 22, wie gestrichelt dargestellt, mit dem ersten Auslasskanal 21 verbindbar ist, so dass mittels der Antriebspumpe 7 die Arbeitspumpe 2 antreibbar ist.

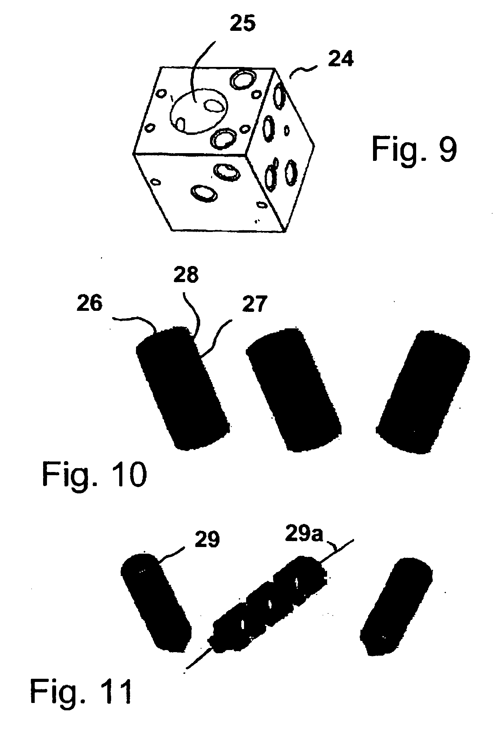

Fig. 9 zeigt den Anschlussblock 24, der eine zylindrische Bohrung 25 aufweist, in die ein in Fig. 10 dargestellter Ventilzylinder 26 dicht einsetzbar ist. Zudem sind in dem Anschlussblock 24 Öffnungen dargestellt, in denen Ein- und Auslasskanäle bzw. Umschaltkanäle münden.

Die entsprechenden Kanäle können innerhalb des Anschlussblocks 24 gerade oder gebogen bzw. abgewinkelt verlaufen, und zwar sowohl innerhalb einer Ebene auf gleichbleibender axialer Höhe in Bezug auf die Drehachse des Steuerzapfens oder auch in axialer Richtung.

Die Fig. 10 zeigt in drei Ansichten aus unterschiedlichen Blickwinkeln den Ventilzylinder 26, der als

Hohlzylinder ausgebildet ist und Nuten 27 zur Aufnahme von Dichtringen aufweist, die zur Abdichtung des Ventilzylinders im Anschlussblock 24 dienen. Zudem weist der Ventilzylinder 26 Öffnungen 28 auf, die den Hohlzylinder durchsetzen und die äußere Mantelfläche mit der inneren Mantelfläche in radialer Richtung verbinden. Dabei sind Öffnungen in drei unterschiedlichen axialen Höhen, bezogen auf die Längsachse des Ventilzylinders, vorgesehen. In der mittleren axialen Höhe sind dabei zwei Öffnungen vorgesehen, die einander diametral gegenüberliegen. In der ersten und zweiten axialen Höhe, d. h. an den beiden Enden des Ventilzylinders, sind jeweils drei Öffnungen vorgesehen, die gegeneinander um 90° am Umfang versetzt sind.

In der Fig. 11 ist ein Steuerzapfen 29 in drei dreidimensionalen Darstellungen aus unterschiedlichen Blickwinkeln dargestellt, wobei in der mittleren Darstellung die Durchgangsrichtung dreier Durchgangskanäle sichtbar ist, die den Steuerzapfen jeweils quer vollständig durchsetzen. Zudem sind in der ersten und der zweiten axialen Höhe zusätzlich quer verlaufende Bohrungen angeordnet, die in den vorgenannten Kanälen münden. Es ergeben sich in der ersten und zweiten axialen Höhe jeweils drei Öffnungen/Mündungen der Durchgangskanäle, die beispielsweise um 90° am Umfang des Steuerzapfens versetzt sind.

Zwischen den drei verschiedenen Ebenen, in denen Durchgangskanäle angeordnet sind, sind radial umlaufende Nuten dargestellt, die zur Aufnahme von Dichtringen dienen, welche nach dem Einschieben des Steuerzapfens in den Ventilzylinder 26 zur Abdichtung dienen.

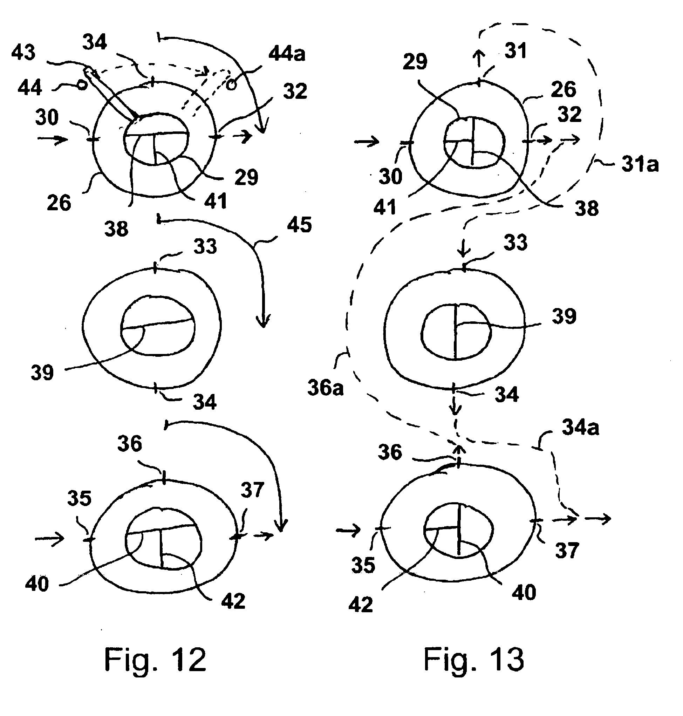

In der Fig. 12 sind untereinander drei Querschnitte durch den Steuerzapfen 29 und den diesen umgebenden Ventilzylinder 26 von oben nach unten in der ersten, dritten (mittleren) und zweiten axialen Höhe dargestellt, wobei diese beiden Elemente in dem Anschlussblock 24 gedacht sind. Der Ventilzylinder 26 ist lediglich als Kreislinie symbolisch dargestellt, mit den entsprechenden radialen Öffnungen, die jeweils nur als kurze Striche 30, 31, 32 dargestellt sind. Entsprechende Kanäle sind in dem zweiten Schnitt mit 33, 34 und in dem dritten Schnitt mit 35, 36, 37 bezeichnet.

Die Durchgangskanäle in den verschiedenen axialen Höhen des Steuerzapfens sind mit 38, 39, 40 (durchgehende Querbohrungen) sowie 41, 42 (Abzweigkanäle) bezeichnet. Die Fig. 12 zeigt eine Winkelstellung des Steuerzapfens 29, in der ein erster Einlasskanal 30 mittels des Durchgangskanals 38 in dem Steuerzapfen mit einem ersten Auslasskanal 32 direkt verbunden ist. Der Abzweigkanal 41 endet blind und spielt in dieser Winkelstellung keine Rolle.

In dieser Winkelstellung des Steuerzapfens 29 ist ein mit dem Steuerzapfen 29 verbundener Hebel 43 an einen als Zapfen ausgebildeten Anschlag 44 angelehnt, um die Winkelstellung zu definieren.

Gleichzeitig ist ein Umschaltkanal, der in den Öffnungen 33, 34 des mittleren Schnitts der Fig. 12 endet, unterbrochen, da keine entsprechenden Durchgangskanäle in dem Zapfen 29 existieren, die in dieser Schaltstellung geeignet positioniert sind.

In dem dritten Schnitt der Fig. 12 ist dargestellt, dass auf der zweiten axialen Höhe der zweite Einlasskanal 35 mit dem zweiten Auslasskanal 37 direkt mittels des Durchgangskanals 40 verbunden ist. Der Abzweigkanal 42 endet in dieser Schaltstellung ebenfalls blind.

Die Öffnungen 31, 36 in dem Ventilzylinder und die entsprechenden weiterführenden Kanäle im Anschlussblock haben in dieser Schaltstellung ebenfalls keine Funktion.

Diese Schaltstellung kann auch als Grundschaltstellung betrachtet werden, in der die Eingangskanäle mit den entsprechenden Ausgangskanälen direkt, auf kürzestem Wege und mit dem geringstmöglichen Strömungswiderstand verbunden sind, so dass die Antriebspumpen 6, 7 jeweils mit ihren Arbeitspumpen 2, 3 durchverbunden sind.

Das Ventil 8 wird dadurch geschaltet, dass, wie in der Fig. 12 angedeutet, der Steuerzapfen 29 in Richtung des Pfeils 45 um 90° im Uhrzeigersinn gedreht wird. Dabei sind auch andere Winkelstellungen denkbar, wobei dann die Winkel zwischen den Durchgangskanälen, Abzweigkanälen und den Ein- und Ausgangskanälen entsprechend angepasst werden müssen.

Die um 90° gedrehte Schaltstellung ist in der Fig. 13 dargestellt. Zunächst wird der obere Querschnitt in der Fig. 13 betrachtet. Der Durchgangskanal 38 verbindet nun nicht mehr den ersten Einlasskanal 30 mit dem ersten Auslasskanal 32. Vielmehr wird der Einlasskanal 30 mit der Öffnung 31 in dem Ventilzylinder verbunden, in die ein Umschaltkanal 31a mündet. Der Umschaltkanal 31a verläuft in dem Anschlussblock 24 wenigstens zum Teil auch in axialer Richtung des Steuerzapfens und verbindet die Öffnung 31 auf der ersten axialen Höhe mit der Öffnung 33 auf der mittleren axialen Höhe, wie im mittleren Querschnitt der Fig. 13 dargestellt. Die Öffnung 33 im Ventilzylinder ist in der in Fig. 13 dargestellten Schaltstellung fluchtend mit dem Durchgangskanal 39 des Steuerzapfens ausgerichtet, so dass der Umschaltkanal 31a mit dem Umschaltkanal 34a, der von der Öffnung 34 ausgeht und zu dem zweiten Auslasskanal 37 führt, verbunden ist. Auf diese Weise ist der erste Einlasskanal 30 mit dem zweiten Auslasskanal 37 verbunden. Gleichzeitig ist, wie im unteren Querschnitt der Fig. 13 sichtbar wird, der zweite Auslasskanal 37 nicht mehr über den Steuerzapfen mit dem zweiten Einlasskanal 35 verbunden. In dieser Stellung des Steuerzapfens ist vielmehr der zweite Einlasskanal 35 mittels des Abzweigkanals 42 und eines Teils des Durchgangskanals 40 mit der Öffnung 36 und dem Umschaltkanal 36a verbunden. Der Umschaltkanal 36a seinerseits ist mit dem ersten Auslasskanal 32 verbunden.

In der mittels Fig. 13 dargestellten Schaltstellung ist damit das 2x2-Wege-Kreuzventil vollständig umgeschaltet, so dass die Eingangskanäle wechselseitig auf die Auslasskanäle umgeschaltet sind.

Fällt nun eine der Antriebspumpen 6, 7, die mit den Einlasskanälen 30, 35 verbunden sind, aus, so kann die verbleibende Antriebspumpe wechselseitig durch Umschaltung zwischen den in Fig. 12 und Fig. 13 dargestellten Positionen auf die beiden Auslasskanäle 32, 37 und damit auf die mit diesen verbundenen Arbeitspumpen umgeschaltet werden.

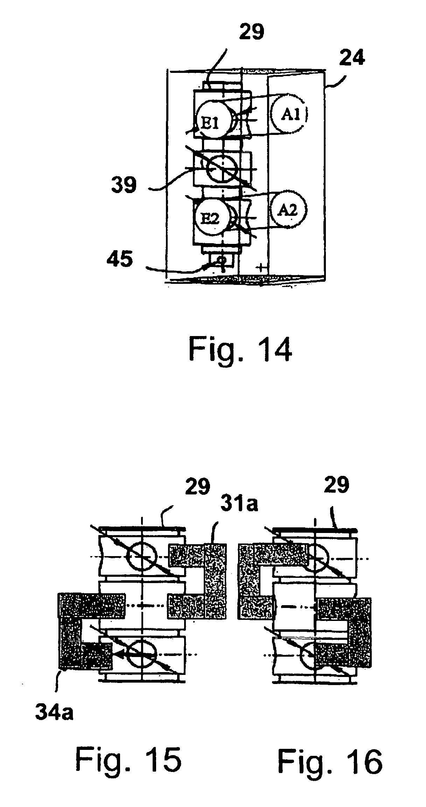

Zur Abrundung ist in der Fig. 14 der Anschlussblock 24 in durchbrochener Darstellung gezeigt, wobei unter Weglassung des Ventilzylinders der Steuerzapfen 29 dargestellt ist. Mit E1 ist der erste Eingangskanal bezeichnet, mit A1 der erste Ausgangskanal, mit E2 der zweite Eingangskanal und mit A2 der zweite Ausgangskanal. Zudem ist der Durchgangskanal 39 dargestellt.

Am unteren Ende des Steuerzapfens 29 ist ein Formkörper 45 dargestellt, auf dem ein nicht gezeigter Permanentmagnet befestigt ist, der mittels eines Elektromagneten rotierend antreibbar ist, um den Steuerzapfen zwischen den Schaltstellungen zu bewegen.

Fig. 15 zeigt in einer schematischen Ansicht den Steuerzapfen 29 mit Umschaltkanälen 31a, 34a, die im Anschlussblock verlaufen, sowie Fig. 16 die entsprechende Darstellung in der entgegengesetzten Blickrichtung.

Durch die dargestellte Ausführungsform eines 2x2-Wege-Kreuzventils kann ein zuverlässig schaltbares Ventil zur Verwendung bei pneumatisch angesteuerten Blutpumpen geschaffen werden, das auch die erforderlichen Standzeiten aufweist. Reibung und Verschleiß können beispielsweise dadurch optimiert werden, dass bei entsprechener Maßtoleranz die Oberflächen auf Keramikbasis mit Aluminium- oder Elematat-Beschichtung versehen sind.

## Patentansprüche

1. Ventil zur Steuerung eines Fluidflusses mit einem Anschlussblock (24), der eine zylindersymmetrische Ausnehmung (25) aufweist, wobei in der Mantelfläche der Ausnehmung mindestens ein erster und ein zweiter Eingangskanal (20, 22, 30, 35) sowie ein erster und ein zweiter Ausgangskanal (21, 23, 32, 37) münden, sowie mit einem in die Ausnehmung eingepassten, um eine Drehachse (29a) drehbaren Steuerzapfen (29), der wenigstens zwei Durchgangskanäle (38, 39, 40, 41, 42) aufweist und in Abhängigkeit von seiner Winkelstellung verschiedene der Eingangs- und Ausgangskanäle des Anschlussblocks miteinander verbindet, wobei ein erster Durchgangskanal (38, 41) in dem Zapfen bezüglich der Drehachse ausschließlich in einer ersten axialen Höhe mündende Kanäle (30, 31, 32) und ein zweiter Durchgangskanal (40, 42) ausschließlich in einer zweiten axialen Höhe mündende Kanäle (35, 36, 37) miteinander verbindet und wobei der Anschlussblock (24) einen Ventilhohlzylinder (26) aufweist, der den Zapfen (29) aufnimmt und Kanäle aufweist, die jeweils einen Teil der Eingangs-, Ausgangs- und Durchgangskanäle bilden, sowie Dichtungen zum Abdichten der Kanäle zwischen dem Zapfen und dem Anschlussblock.

2. Ventil nach Anspruch 1, **dadurch gekennzeichnet, dass** wenigstens ein Durchgangskanal (3, 41, 40, 42), insbesondere der erste und der zweite Durchgangskanal, quer durch den Zapfen (29) zwischen je einer ersten und einer zweiten diametral einander gegenüberliegenden Zapfenöffnung verläuft und einen Abzweigkanal (41, 42) aufweist, der an einer dritten Zapfenöffnung endet.

3. Ventil nach Anspruch 1 oder 2, **dadurch gekennzeichnet, dass** in dem Anschlussblock (24) wenigstens ein erster, in der ersten axialen Höhe in der Mantelfläche der Ausnehmung mündender Umschaltkanal (36a) vorgesehen ist, der diese Mündung mit einem in der zweiten axialen Höhe mündenden Kanal (36) verbindet.

4. Ventil nach Anspruch 1, 2 oder 3, **dadurch gekennzeichnet, dass** in dem Anschlussblock (24) wenigstens ein zweiter, in der zweiten axialen Höhe in der Mantelfläche der Ausnehmung mündender Umschaltkanal (31a, 34a) vorgesehen ist, der diese Mündung mit einem in der ersten axialen Höhe mündenden Kanal verbindet.

5. Ventil nach einem der Ansprüche 1 bis 4, **dadurch gekennzeichnet, dass** der Zapfen einen dritten Durchgangskanal (39) aufweist.

6. Ventil nach Anspruch 5, **dadurch gekennzeichnet, dass** der dritte Durchgangskanal in Abhängigkeit von der Winkelstellung des Zapfens zwei Abschnitte eines Umschaltkanals (31a, 34a) miteinander verbindet.

7. Ventil nach Anspruch 5 oder 6, **dadurch gekennzeichnet, dass** der dritte Durchgangskanal (39) auf einer axialen Höhe zwischen der ersten und der zweiten axialen Höhe verläuft.

8. Ventil nach Anspruch 1 oder einem der folgenden, **dadurch gekennzeichnet, dass** der Steuerzapfen (29) an einem seiner Enden mit einem Magneten verbunden ist, der einen Teil eines Drehantriebes bildet.

9. Ventil nach Anspruch 1 oder einem der folgenden, **dadurch gekennzeichnet, dass** mechanische Anschläge (44, 44a) zwischen dem Zapfen (29) und dem Anschlussblock (24) vorgesehen sind, die zwei Endwinkelstellungen des Zapfens definieren.

10. Pumpensystem mit zwei Antriebspumpen (6,7), die mittels Fluidleitungen (4, 5) mit jeweils einer Arbeitspumpe (2, 3) zur Förderung einer Flüssigkeit, insbesondere Blut verbindbar sind, wobei beide Fluidleitungen ein gemeinsames Ventil (8) nach einem der Ansprüche 1 bis 9 durchlaufen und das Ventil (8) als 2x2-Wege-Kreuzventil ausgebildet ist.

11. Verfahren zum Betrieb eines Pumpensystems mit einem Ventil gemäß einem der Ansprüche 1 bis 9, das mit wenigstens einer arbeitsfähigen Antriebspumpe (6, 7) und mit zwei Arbeitspumpen (2, 3) verbunden und als 2x2-Wege-Kreuzventil ausgebildet ist, **dadurch gekennzeichnet, dass** das Ventil eine Antriebspumpe (6, 7) abwechselnd mit je einer der Arbeitspumpen (2, 3) verbindet.

## Claims

1. A valve for the control of a fluid flow, with a connection block (24) which comprises a cylinder-symmetrical recess (25), wherein at least one first and a second entry channel (20, 22, 30, 35) as well as a first and a second exit channel (21, 23, 32, 37) run out in the peripheral surface of the recess, as well as with a control pin (29) which is fitted into the recess, is rotatable about a rotation axis (29a), comprises at least two through-channels (38, 39, 40, 41, 42) and connects different ones of the entry and exit channels of the connection block to one another depending on its angular position, wherein a first through-channel (38, 41) in the pin exclusively connects channels (30, 31, 32) running out at a first axial height with respect to the rotation axis to one another, and a second through-channel (40, 42) exclusively connects channels (35, 36, 37) running out at a second axial height, to one another, and wherein the connection block (24) comprises a valve hollow cylinder (26) which receives the pin (29) and comprises channels each of which form a part of the entry, exit and through-channels, as well as seals for sealing the channels between the pin and the connection block.

2. A valve according to claim 1, **characterised in that** at least one through-channel (3, 41, 40, 42), in particular the first and the second through-channel, runs transversely through the pin (29) between in each case a first and a second pin opening lying diametrically opposite one another, and comprises a branch-off channel (41, 42) which ends at a third pin opening.

3. A valve according to claim 1 or 2, **characterised in that** in the connection block (24), at least one first switch-over channel (36a) running out at the first axial height in the peripheral surface of the recess is provided, which connects this run-out to a channel (36) running out at the second axial height.

4. A valve according to claim 1, 2 or 3, **characterised in that** at least one second switch-over channel (31a, 34a) running out at the second axial height in the peripheral surface of the recess is provided in the connection block (24), which connects this run-out to a channel running out at the first axial height.

5. A valve according to one of claims 1 to 4, **characterised in that** pin comprises a third through-channel (39).

6. A valve according to claim 5, **characterised in that** the third through-channel connects two sections of the switch-over channel (31a, 34a) to one another, depending on the angular position of the pin.

7. A valve according to claim 5 or 6, **characterised in that** the third through-channel (39) runs at an axial height between the first and the second axial height.

8. A valve according to claim 1 or one of the following ones, **characterised in that** the control pin (29) is connected at one of its ends to a magnet which forms a part of a rotary drive.

9. A valve according to claim 1 or one of the following ones, **characterised in that** mechanical abutments (44, 44a) are provided between the pin (29) and the connection block (24), which define two end angle positions of the pin.

10. A pump system with two drive pumps (6, 7) which are connectable by way of fluid conduits (4, 5) in each case to a working pump (2, 3) for delivering a fluid, in particular blood, wherein both fluid conduits run through a common valve (8) according to one of the claims 1 to 9, and the valve (8) is designed as a 2x2-way cross-over valve.

11. A method for the operation of a pump system with a valve according to one of the claims 1 to 9, which is connected to at least one operational drive pump (6, 7) and to two working pumps (2, 3) and is designed as a 2x2-way cross-over valve, **characterised in that** the valve alternatingly connects a drive pump (6, 7) in each case to one of the working pumps (2, 3).

## Revendications

1. Soupape pour la commande d'un flux de fluide, avec un bloc de connexion (24) comportant un évidement (25) cylindriquement symétrique, dans laquelle au moins un premier et un deuxième canal d'entrée (20, 22, 30, 35) ainsi qu'un premier et un deuxième canal de sortie (21, 23, 32, 37) débouchent sur la surface d'enveloppe de l'évidement, ainsi qu'avec un téton de commande (29) rotatif autour d'un axe de rotation (29a) et ajusté dans l'évidement, lequel comporte au moins deux canaux de passage (38, 39, 40, 41, 42) et relie entre eux différents canaux d'entrée et de sortie du bloc de connexion, en fonction de sa position angulaire, dans laquelle un premier canal de passage (38, 41) dans le téton relie entre eux exclusivement les canaux (30, 31, 32) débouchant à une première hauteur axiale par rapport à l'axe de rotation, et un deuxième canal de passage (40, 42) relie entre eux exclusivement les canaux (35, 36, 37) débouchant à une deuxième hauteur axiale, et dans laquelle le bloc de connexion (24) comporte un cylindre creux de soupape (26) accueillant le téton (29) et comportant des canaux formant respectivement une partie des canaux d'entrée, de sortie et de passage, ainsi que des dispositifs d'étanchéité pour l'étanchéité des canaux entre le téton et le bloc de connexion.

2. Soupape selon la revendication 1, **caractérisée en ce qu'**au moins un canal de passage (3, 41, 40, 42), en particulier le premier et le deuxième canal de passage, s'étend transversalement au téton (29), respectivement entre une première ouverture de téton et une deuxième ouverture de téton diamétralement opposée, et comporte un canal de déviation (41, 42) finissant en une troisième ouverture de téton.

3. Soupape selon la revendication 1 ou 2, **caractérisée en ce que** dans le bloc de connexion (24), il est prévu au moins un premier canal de commutation (36a) débouchant sur la surface d'enveloppe à la première hauteur axiale, lequel relie cette embouchure avec un canal (36) débouchant à la deuxième hauteur axiale.

4. Soupape selon la revendication 1, 2 ou 3, **caractérisée en ce que** dans le bloc de connexion (24), il est prévu au moins un deuxième canal de commutation (31a, 34a) débouchant sur la surface d'enveloppe à une deuxième hauteur axiale, lequel relie cette embouchure à un canal débouchant à la première hauteur axiale.

5. Soupape selon l'une des revendications 1 à 4, **caractérisée en ce que** le téton comporte un troisième canal de passage (39).

6. Soupape selon la revendication 5, **caractérisée en ce que** le troisième canal de passage relie entre elles deux sections d'un canal de commutation (31a, 34a) en fonction de la position angulaire du téton.

7. Soupape selon la revendication 5 ou 6, **caractérisée en ce que** le troisième canal de passage (39) s'étend à une hauteur axiale entre la première et la deuxième hauteur axiale.

8. Soupape selon la revendication 1 ou l'une des suivantes, **caractérisée en ce que** le téton de commande (29) est relié à un aimant par l'une de ses extrémité, lequel forme une partie d'un entraînement rotatif.

9. Soupape selon la revendication 1 ou l'une des suivantes, **caractérisée en ce que** des butées mécaniques (44, 44a) sont prévues entre le téton (29) et le bloc de connexion (24), lesquelles définissent deux positions angulaires finales du téton.

10. Système de pompe avec deux pompes d'entraînement (6, 7) pouvant être reliées respectivement à une pompe de travail (2, 3) par des conduites de fluide (4, 5) pour transporter un liquide, en particulier du sang, dans lequel les deux conduites de fluide traversent une soupape commune (8) selon l'une des revendications 1 à 9, et la soupape (8) est conçue comme une soupape en croix à 2 x 2 voies.

11. Procédé pour le fonctionnement d'un système de pompe avec une soupape selon l'une des revendications 1 à 9, laquelle est reliée à au moins une pompe d'entraînement (6, 7) opérationnelle et à deux pompes de travail (2, 3), et conçue comme une soupape en croix à 2 x 2 voies, **caractérisé en ce que** la soupape relie une pompe d'entraînement (6, 7) en alternance respectivement à l'une des pompes de travail (2, 3).
